# EUROPEAN PATENT SPECIFICATION

(11) **EP 3 119 388 B1**
(45) Date of publication and mention of the grant of the patent: **17.06.2020**
(21) Application number: 15765060.7
(22) Date of filing: 20.03.2015
(51) Int. Cl.: A61K 31/194, A61P 1/16, A61P 31/12, A61P 31/18, A61P 1/00, A61P 3/06

(54) **CARBOXY-CYCLOPROPYL UNDECANOL COMPOUNDS FOR TREATMENT OF LIVER DISEASE AND OTHER MEDICAL DISORDERS**
CARBOXY-CYCLOPROPYL-UNDECANOLVERBINDUNGEN ZUR BEHANDLUNG VON LEBERERKRANKUNGEN UND ANDERER ERKRANKUNGEN
COMPOSÉS CARBOXY-CYCLOPROPYL UNDÉCANOL POUR LE TRAITEMENT DE MALADIE HÉPATIQUE ET D'AUTRES TROUBLES MÉDICAUX

(30) Priority: 20.03.2014 US 201461968082 P
(43) Date of publication of application: 25.01.2017
(73) Proprietor: Esperion Therapeutics, Inc., Ann Arbor, MI 48108 (US)
(72) Inventor: HANSELMAN, Jeffrey, C., Chelsea, MI 48118 (US); SRIVASTAVA, Rai Ajit, K., Churchville, PA 18966 (US); NEWTON, Roger, S., Ann Arbor, MI 48105 (US)
(74) Representative: Callaghan, Dayle Anne
(86) International application number: PCT/US2015/021677
(87) International publication number: WO 2015/143276

(56) References cited:
- WO-A1-2012/040177
- WO-A2-2004/067489
- STEPHEN L. PINKOSKY ET AL: "AMP-activated protein kinase and ATP-citrate lyase are two distinct molecular targets for ETC-1002, a novel small molecule regulator of lipid and carbohydrate metabolism", JOURNAL OF LIPID RESEARCH, vol. 54, no. 1, 1 January 2013 (2013-01-01), pages 134-151, XP55413416, US ISSN: 0022-2275, DOI: 10.1194/jlr.M030528

## Description

### CROSS-REFERENCE TO RELATED APPLICATIONS

This application claims the benefit of and priority to United States Provisional Patent Application serial number 61/968,082, filed March 20, 2014.

### FIELD OF THE INVENTION

The invention provides carboxy-cyclopropyl undecanol compounds and pharmaceutically acceptable salts thereof for use in treating liver disease and other medical disorders associated with elevated lipid levels in the liver.

### BACKGROUND

Fatty liver disorders, also known as fatty liver or fatty liver disease (FLD), relate to a condition where large vacuoles of triglyceride fat accumulate in liver cells. In its early form, FLD is often relatively benign and may be reversible, but it can lead to more serious conditions. FLD may be categorized into two separate conditions: alcoholic FLD (also known as alcoholic steatosis; caused at least in part by alcohol abuse) and non-alcoholic FLD (NAFLD; not caused by alcohol abuse). Both conditions often show micro-vesicular and macro-vesicular fatty changes at different stages of the disease. Accumulation of fat may also be accompanied by a progressive inflammation of the liver (hepatitis), called steatohepatitis. Steatohepatitis may be further specified as alcoholic steatohepatitis and non-alcoholic steatohepatitis (NASH). Non-alcoholic fatty liver disease-associated cirrhosis is the most severe form of the disease and is typically characterized by liver inflammation that leads to scarring of liver tissue.

Non-alcoholic fatty liver disease and non-alcoholic steatohepatitis are common causes of chronic liver disease in the adult population and represent crucial risk factors for progression to liver cirrhosis, hepatocellular carcinoma, and liver failure. Some experts estimate that about two-thirds of obese adults and half of obese children may have fatty liver. The increased incidence of NAFLD and NASH may be linked to the increasing incidence of obesity in the United States. NASH can cause scarring and hardening of the liver, leading to cirrhosis and eventually to hepatocellular carcinoma and liver failure.

Despite the increasing incidence of NASH and other medical disorders associated with elevated lipid levels in the liver, medical treatment options are quite limited. The present invention addresses this need and provides other related advantages.

### SUMMARY

The invention provides carboxy-cyclopropyl undecanol compounds and pharmaceutically acceptable salts thereof for use in treating liver disease and other medical disorders associated with elevated lipid levels in the liver. Particularly preferred embodiments provide the compound 1,1 1-bis-(1-carboxy-cyclopropyl)-undecan-6-ol or a pharmaceutically acceptable salt thereof for use in treating steatohepatitis, such as nonalcoholic steatohepatitis. The compounds provide benefits to both pediatric and adult patients that may suffer from medical disorders described herein, such as nonalcoholic steatohepatitis.

Accordingly, one aspect of the invention provides a compound for use in treating a liver disorder selected from the group consisting of steatohepatitis, alcoholic liver disease, fatty liver, liver steatosis, liver cirrhosis, liver fibrosis, and acute fatty liver of pregnancy. Also described is administering to a patient in need thereof a therapeutically effective amount of a compound selected from the group consisting of 1,1 1-bis-(1-carboxy-cyclopropyl)-undecan-6-ol and a pharmaceutically acceptable salt thereof to treat the disorder.

Another aspect of the invention provides a compound for use in treating a disorder selected from the group consisting of lipodystrophy, lysosomal acid lipase deficiency, and a glycogen storage disease. Also described is administering to a patient in need thereof a therapeutically effective amount of a compound selected from the group consisting of 1,11-bis-(1-carboxy-cyclopropyl)-undecan-6-ol and a pharmaceutically acceptable salt thereof to treat the disorder.

Yet another aspect of the invention provides a compound for use in treating a disorder selected from the group consisting of hepatitis C, an infection by human immunodeficiency virus, an alpha 1-antitrypsin deficiency, Bassen-Kornzweig syndrome, hypobetalipoproteinemia, Celiac disease, Wilson disease, and Weber-Christian syndrome. Also described is administering to a patient in need thereof a therapeutically effective amount of a compound selected from the group consisting of 1,1 1-bis-(1-carboxy-cyclopropyl)-undecan-6-ol and a pharmaceutically acceptable salt thereof to treat the disorder.

Yet another aspect of the invention provides a compound for use in treating a condition selected from the group consisting of toxic liver injury, total parenteral nutrition, severe surgical weight loss, environmental toxicity, malnutrition, and starvation. Also described is administering to a patient in need thereof a therapeutically effective amount of a compound selected from the group consisting of 1,11-bis-(1-carboxy-cyclopropyl)-undecan-6-ol and a pharmaceutically acceptable salt thereof to treat the condition.

Various aspects and embodiments of the invention are described in further detail below.

### BRIEF DESCRIPTION OF THE DRAWINGS

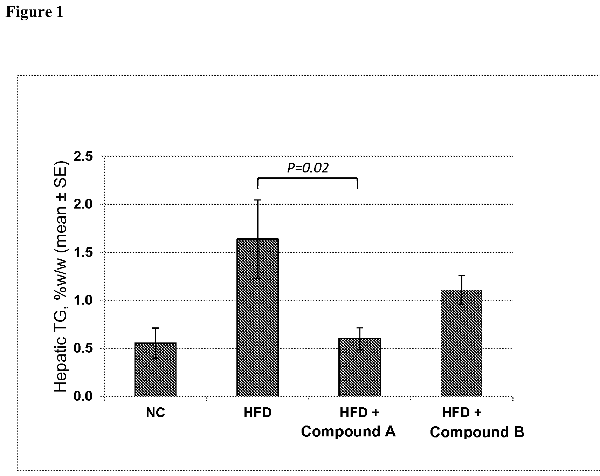

**FIGURE 1** shows hepatic triglyceride (TG) content of C57BL/6N male mice fed either a normal chow diet (NC); high-fat diet (HFD); high-fat diet + Compound A; or high-fat diet + Compound B, as explained in Example 1. Three week high-fat feeding resulted in increased hepatic TG levels compared to normal chow fed mice (1.6 vs. 0.6 % w/w, P = 0.03). Addition of Compound A to the high-fat diet for the final 2 weeks attenuated accumulation of hepatic TG significantly compared to high-fat feeding alone (0.6 vs. 1.6 % w/w, P = 0.02) and was more efficacious than Compound B (1.1 % w/w).

### DETAILED DESCRIPTION OF THE INVENTION

The invention provides carboxy-cyclopropyl undecanol compounds and pharmaceutically acceptable salts thereof for use in treating liver disease and other medical disorders associated with elevated lipid levels in the liver. Also described is methods of using compositions containing such compounds and/or medical kits to treat medical disorders in a patient. Particularly preferred embodiments provide the compound 1,11-bis-(1-carboxy-cyclopropyl)-undecan-6-ol or a pharmaceutically acceptable salt thereof for use in treating steatohepatitis, such as nonalcoholic steatohepatitis. The compound 1,11-bis-(1-carboxy-cyclopropyl)-undecan-6-ol has the following chemical formula (hereinafter designated "Compound A"):

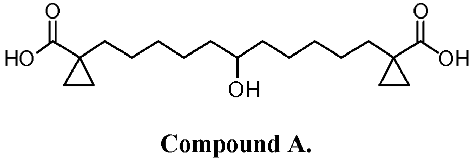

Various aspects of the invention are set forth below in sections; however, aspects of the invention described in one particular section are not to be limited to any particular section.

### I. THERAPEUTIC APPLICATIONS

The invention provides carboxy-cyclopropyl undecanol compounds and pharmaceutically acceptable salts thereof for use in treating liver disease and other medical disorders associated with elevated lipid levels in the liver. Also described is the use of carboxy-cyclopropyl undecanol compounds described herein as stand-alone therapeutic agents, and/or as part of a combination therapy with another therapeutic agent.

### Treating Liver Disorders

One aspect of the invention provides a compound for use in treating a liver disorder selected from the group consisting of steatohepatitis, alcoholic liver disease, fatty liver, liver steatosis, liver cirrhosis, liver fibrosis, and acute fatty liver of pregnancy. Also described is administering to a patient in need thereof a therapeutically effective amount of a compound selected from the group consisting of 1,1 1-bis-(1-carboxy-cyclopropyl)-undecan-6-ol and a pharmaceutically acceptable salt thereof to treat the disorder.

In certain embodiments, the disorder is steatohepatitis. In certain other embodiments, the steatohepatitis is nonalcoholic steatohepatitis. In certain other embodiments, the steatohepatitis is nonalcoholic fatty liver disease.

In certain embodiments, the disorder is alcoholic liver disease.

In certain embodiments, the disorder is fatty liver.

In certain embodiments, the disorder is liver steatosis, liver cirrhosis, or liver fibrosis.

In certain embodiments, the disorder is acute fatty liver of pregnancy.

In certain embodiments, the compound is 1,11-bis-(1-carboxy-cyclopropyl)-undecan-6-ol.

In certain embodiments, the patient is an adult human.

### Treating Lipodystrophy and Other Disorders

Another aspect of the invention provides a compound for use in treating a disorder selected from the group consisting of lipodystrophy, lysosomal acid lipase deficiency, and a glycogen storage disease. Also described is administering to a patient in need thereof a therapeutically effective amount of a compound selected from the group consisting of 1,11-bis-(1-carboxy-cyclopropyl)-undecan-6-ol and a pharmaceutically acceptable salt thereof to treat the disorder.

In certain embodiments, the compound is 1,1 1-bis-(1-carboxy-cyclopropyl)-undecan-6-ol.

In certain embodiments, the patient is an adult human.

### Treating Hepatitis C Infection and Other Disorders

Another aspect of the invention provides a compound for use in treating a disorder selected from the group consisting of hepatitis C, an infection by human immunodeficiency virus, an alpha 1-antitrypsin deficiency, Bassen-Kornzweig syndrome, hypobetalipoproteinemia, Celiac disease, Wilson disease, and Weber-Christian syndrome. Also described is administering to a patient in need thereof a therapeutically effective amount of a compound selected from the group consisting of 1,11-bis-(1-carboxy-cyclopropyl)-undecan-6-ol and a pharmaceutically acceptable salt thereof to treat the disorder.

In certain embodiments, the disorder is hepatitis C. In certain other embodiments, the disorder is an infection by human immunodeficiency virus. In certain other embodiments, the disorder is an alpha 1-antitrypsin deficiency. In certain other embodiments, the disorder is Bassen-Kornzweig syndrome. In certain other embodiments, the disorder is hypobetalipoproteinemia. In certain other embodiments, the disorder is Celiac disease or Wilson disease. In certain other embodiments, the disorder is Weber-Christian syndrome.

In certain embodiments, the compound is 1,11-bis-(1-carboxy-cyclopropyl)-undecan-6-ol.

In certain embodiments, the patient is an adult human.

### Treating Toxic Liver Injury and Other Conditions

Another aspect of the invention provides a compound for use in treating a condition selected from the group consisting of toxic liver injury, total parenteral nutrition, severe surgical weight loss, environmental toxicity, malnutrition, and starvation. Also described is administering to a patient in need thereof a therapeutically effective amount of a compound selected from the group consisting of 1,11-bis-(1-carboxy-cyclopropyl)-undecan-6-ol and a pharmaceutically acceptable salt thereof to treat the condition.

In certain embodiments, the condition is toxic liver injury. In certain embodiments, the condition is total parenteral nutrition or severe surgical weight loss. In certain embodiments, the condition is environmental toxicity. In certain embodiments, the condition is malnutrition or starvation.

In certain embodiments, the compound is 1,11-bis-(1-carboxy-cyclopropyl)-undecan-6-ol.

In certain embodiments, the patient is an adult human.

The description above describes multiple embodiments relating to use of certain carboxy-cyclopropyl undecanol compounds or pharmaceutically acceptable salts thereof in treating various diseases, disorders, and conditions. The patent application specifically contemplates all combinations of the embodiments. For example, the patent application contemplates treating nonalcoholic steatohepatitis by administering to a patient in need thereof a therapeutically effective amount of 1,11-bis-(1-carboxy-cyclopropyl)-undecan-6-ol to thereby treat nonalcoholic steatohepatitis.

### Exemplary Procedures for Patient Identification

Procedures for identifying patients suffering from one or more of the medical disorders described herein are described in the literature and/or can be determined by medical personnel according to routine medical procedures. For example, diagnostic methods based on identification of biomarkers have been described for predicting, diagnosing, or staging NAFLD. Certain reports indicate that those with NAFLD at risk for progression of hepatic steatosis to NASH can be identified based on unexplained increases to >2.5 times the upper limit of AST levels, ALT levels, or both, excessive body weight, type 2 diabetes, and unexplained hepatomegaly. Other markers include increased alkaline phosphatase, elevated ferritin levels, ALT concentrations exceeding AST levels, and unexplained increases in serum aminotransferase levels in overweight and obese individuals (Grattagliano et al., Can. Fam. Physician 2007, 53, 857-86).

While determination of biomarker levels can be used to predict those at risk of developing NAFLD or diagnose those with NAFLD, these marker assays can be supplemented with histological assessments (e.g., liver biopsies) or non-invasive imaging modalities. Non-invasive imaging modalities known in the art for assessing NAFLD include, but are not limited to, magnetic resonance imaging (MRI), diffusion weighted MRI, and ultrasound elastography or MRI elastography. Such non-invasive imaging modalities provide a useful and painless alternative for determining the presence of NAFLD and staging NAFLD progression. Other non-invasive methods for determining mammals at risk for NAFLD and/or determining whether a mammal has a mild or severe form of NAFLD are disclosed in, e.g., U.S. Patent Publication No. 2009/0220986.

Various genetic disorders are associated with severe hepatic steatosis, such as glycogen storage disease type la, citrin deficiency, and congenital generalized lipodystrophy. Many genomic sequence variants associated with the full spectrum of NAFLD have been identified and can be used to determine those who are at risk of developing the disease (Cohen et al., Science 2011, 332, 1519-23). Such variants include mutations or sequence variants in genes such as ATGL, comparative gene identification-58 (CGI-58), hydroxyacyl-CoA transferases, VLDL, microsomal TG transfer protein, APOB, and PNPLA3. Thus, using routine techniques known in the art (e.g., DNA sequencing, RFLP analysis, and genetic linkage studies), subjects can be identified who are at risk of developing NALFD and can thus be prophylactically treated with a compound of the present invention.

In some embodiments, patients at risk of developing or who have NAFLD are affected by metabolic syndrome. Metabolic syndrome is a collection of risk factors estimated to affect over 50 million Americans. There is currently a lack of well-accepted criteria for diagnosing metabolic syndrome; however, those that are affected typically exhibit abdominal obesity, atherogenic dislipidemia, elevated blood pressure, insulin resistance or glucose intolerance, prothrombotic state and a proinflammatory state.

In some embodiments, the invention is directed to using carboxy-cyclopropyl undecanol compounds or pharmaceutically acceptable salts thereof to treat liver disease and other medical disorders associated with elevated lipid levels in the liver, wherein the mammal in need of such treatment has or is at risk of developing metabolic syndrome. Patients with metabolic syndrome can be identified via a number of established criteria. Exemplary criteria currently used to diagnose metabolic syndrome can be found in, for example, the World Health Organization (criteria established in 1999) (World Health Organization (WHO). Definition, diagnosis and classification of diabetes mellitus and its complications. Report of a WHO consultation, 1999), the Adult Treatment Panel III Report 2001 (Expert panel on detection, evaluation, and treatment of high blood cholesterol in adults. JAMA 2001, 285, 2486-97), the European Group for the Study of IR (EGIR) 1999 (Balkau B et al., Diabet. Med. 1999, 16, 442-3), and the International Diabetes Federation (IDF) consensus on metabolic syndrome.

### Combination Therapy

As indicated above, the invention embraces combination therapy, which includes the administration of a carboxy-cyclopropyl undecanol compound or pharmaceutically acceptable salt thereof (such as Compound A) and a second agent as part of a specific treatment regimen intended to provide the beneficial effect from the co-action of these therapeutic agents. The beneficial effect of the combination may include pharmacokinetic or pharmacodynamic co-action resulting from the combination of therapeutic agents.

Contemplated agents that may be co-administered with a carboxy-cyclopropyl undecanol compound or pharmaceutically acceptable salt thereof in the present methods include antiviral agents (e.g., to treat an underlying hepatitis C infection causing liver disease in the patient), anticancer agents (e.g., to treat hepatocellular carcinoma or other cancer causing liver disease or fatty liver), antioxidants, medications that decrease insulin resistance, or medications that improve lipid metabolism (e.g., treatments for hyperlipidemia).

### II. PHARMACEUTICAL COMPOSITIONS

The invention provides pharmaceutical compositions comprising a carboxy-cyclopropyl undecanol compound or pharmaceutically acceptable salt thereof. In certain embodiments, the pharmaceutical compositions preferably comprise a therapeutically-effective amount of one or more of the carboxy-cyclopropyl undecanol compounds or pharmaceutically acceptable salts thereof described herein, formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets (e.g., those targeted for buccal, sublingual, and/or systemic absorption), boluses, powders, granules, pastes for application to the tongue; (2) parenteral administration by, for example, subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "therapeutically-effective amount" as used herein means that amount of a compound, material, or composition comprising a compound of the present invention which is effective for producing some desired therapeutic effect in at least a sub-population of cells at a reasonable benefit/risk ratio applicable to any medical treatment. In certain embodiments, the sub-population of cells is in an animal. In certain embodiments, the sub-population of cells is in a human.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration.

The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, or most preferably from about 10 percent to about 30 percent.

In certain embodiments, a formulation of the present invention comprises an excipient selected from the group consisting of cyclodextrins, celluloses, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The preparations of the present invention may be given orally, parenterally, topically, or rectally. They are of course given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administrations are preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Preferably, the compounds are administered at about 0.01 mg/kg to about 200 mg/kg, more preferably at about 0.1 mg/kg to about 100 mg/kg, even more preferably at about 0.5 mg/kg to about 50 mg/kg. When the compounds described herein are co-administered with another agent (*e.g.,* as sensitizing agents), the effective amount may be less than when the agent is used alone.

If desired, the effective daily dose of the active compound may be administered as two, three, four, five, six or more sub-doses administered separately at appropriate intervals throughout the day, optionally, in unit dosage forms. Preferred dosing is one administration per day.

### III. KITS FOR USE IN MEDICAL APPLICATIONS

Also described is a kit for treating a disorder. The kit comprises: i) instructions for treating a liver disorder, such as nonalcoholic steatohepatitis; and ii) a carboxy-cyclopropyl undecanol compound or pharmaceutically acceptable salt thereof, such as Compound A. The kit may comprise one or more unit dosage forms containing an amount of a carboxy-cyclopropyl undecanol compound or pharmaceutically acceptable salt thereof, that is effective for treating said liver disorder.

The description above describes methods of using a carboxy-cyclopropyl undecanol compound or pharmaceutically acceptable salt thereof, compositions comprising a carboxy-cyclopropyl undecanol compound or pharmaceutically acceptable salt thereof, and kits. The patent application specifically contemplates all combinations and permutations of the aspects and embodiments.

### IV. DEFINITIONS

To facilitate an understanding of the present invention, a number of terms and phrases are defined below.

The terms "a" and "an" as used herein mean "one or more" and include the plural unless the context is inappropriate.

"Modulating" or "modulate" in the context of the present invention means increasing, decreasing, or otherwise altering, adjusting, varying, changing, enhancing or inhibiting a biological event. Likewise, a "modulator" may be a compound, agent or drug that increases, decreases, alters, adjusts, varies, changes, enhances, or inhibits a biological event.

"Liver disorder" as used herein refers generally to diseases, disorders, and conditions affecting the liver, and may have a wide range of severity encompassing, for example, simple accumulation of fat in the hepatocytes (steatosis), macrovescicular steatosis, periportal and lobular inflammation (steatohepatitis), cirrhosis, fibrosis, liver cancers, and liver failure.

As used herein, the term "fatty liver disease," which is also called fatty liver, refers to a disease leading to liver injury caused by abnormal fat accumulation in liver cells. FLD may arise from a number of sources, including excessive alcohol consumption and metabolic disorders, such as those associated with insulin resistance, obesity, and hypertension.

As used herein, the term "non-alcoholic fatty liver disease" (NAFLD) is intended to refer to the spectrum of disorders resulting from an accumulation of fat in liver cells in individuals with no history of excessive alcohol consumption. In the mildest form, NAFLD refers to hepatic steatosis.

The term "drug-induced liver disease" or "toxic liver injury" is used to describe those instances in which an active agent has caused injury to the liver.

The term "alcoholic liver disease" (also called alcoholic liver injury) as used herein refers to a disease caused by fat accumulation in liver cells caused at least in part by alcohol ingestion. Examples include, but are not limited to, diseases such as alcoholic simple fatty liver, alcoholic steatohepatitis (ASH), alcoholic hepatic fibrosis, alcoholic cirrhosis and the like. It should be noted that alcoholic steatohepatitis is also called alcoholic fatty hepatitis and includes alcoholic hepatic fibrosis.

Acute fatty liver conditions can also arise during pregnancy and can be life-threatening. These conditions are referred to herein as "fatty liver of pregnancy".

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the symbols "R" or "S," depending on the configuration of substituents around the stereogenic carbon atom. The present invention encompasses various stereoisomers of these compounds and mixtures thereof. Stereoisomers include enantiomers and diastereomers. Mixtures of enantiomers or diastereomers may be designated "(±)" in nomenclature, but the skilled artisan will recognize that a structure may denote a chiral center implicitly. It is understood that graphical depictions of chemical structures, e.g., generic chemical structures, encompass all stereoisomeric forms of the specified compounds, unless indicated otherwise.

Individual stereoisomers of compounds of the present invention can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns. Stereoisomeric mixtures can also be resolved into their component stereoisomers by well known methods, such as chiral-phase gas chromatography, chiral-phase high performance liquid chromatography, crystallizing the compound as a chiral salt complex, or crystallizing the compound in a chiral solvent. Stereoisomers can also be obtained from stereomerically-pure intermediates, reagents, and catalysts by well known asymmetric synthetic methods.

The invention also embraces isotopically labeled compounds of the invention which are identical to those recited herein, except that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

As used herein, the terms "subject" and "patient" refer to organisms to be treated by the methods of the present invention. Such organisms are preferably mammals (e.g., murines, simians, equines, bovines, porcines, canines, felines, and the like), and more preferably humans.

As used herein, the term "effective amount" refers to the amount of a compound (e.g., a compound of the present invention) sufficient to effect beneficial or desired results. An effective amount can be administered in one or more administrations, applications or dosages and is not intended to be limited to a particular formulation or administration route. As used herein, the term "treating" includes any effect, e.g., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (e.g., such as an oil/water or water/oil emulsions), and various types of wetting agents. The compositions also can include stabilizers and preservatives. For examples of carriers, stabilizers and adjuvants, see, e.g., Martin, Remington's Pharmaceutical Sciences, 15th Ed., Mack Publ. Co., Easton, PA [1975].

As used herein, the term "pharmaceutically acceptable salt" refers to any pharmaceutically acceptable salt (e.g., acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (e.g., sodium) hydroxides, alkaline earth metal (e.g., magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

Throughout the description, where compositions and kits are described as having, including, or comprising specific components, or where processes and methods are described as having, including, or comprising specific steps, it is contemplated that, additionally, there are compositions and kits of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### EXAMPLES

The invention now being generally described, will be more readily understood by reference to the following examples, which are included merely for purposes of illustration of certain aspects and embodiments of the present invention, and are not intended to limit the invention.

### EXAMPLE 1 - EFFECT OF COMPOUNDS A AND BON HEPATIC TRIGLYCERIDE LEVELS IN C57BL/6N MALE MICE RECEIVING A HIGH-FAT DIET

An assay was performed to determine the effect of administering Compounds A and B to C57BL/6N male mice receiving a high-fat diet. Compound structures, experimental methods, and results of the assay are provided below.

### Compounds

The following test compounds were used in this study:

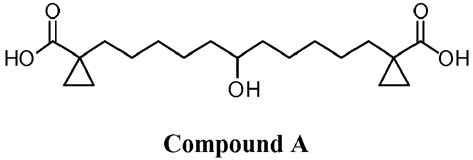

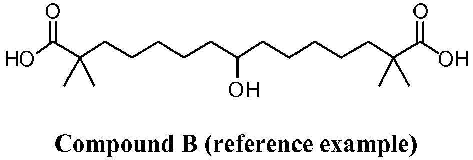

### Experimental Methods

C57BL/6N male mice were obtained from Charles River Laboratories (Montreal, QC, Canada) at 11 weeks of age and singly housed on alpha-dri paper bedding on a normal 12 hour, 6 am-6 pm light-dark cycle. Upon arrival, 27 mice were fed D12492 high fat diets (HFD) containing 60% kcal fat while 9 mice were fed D12450K normal chow diet (NC) for 1 week with free access to water. Mice that had been fed D12492 were allocated into three equally distributed treatment arms of 9 mice each at 12 weeks of age based on 4-hour fasted blood glucose and body weight. One arm continued to receive HFD, one arm was switched to a diet admix consisting of 289 mg of Compound A per 1 kg HFD (HFD + Compound A), and one arm was switched to a diet admix consisting of 289 mg of Compound B per 1kg HFD (HFD + Compound B) as a comparator so that ∼30 mg/kg/day Compound B or Compound A was consumed daily based on an estimated food consumption of ∼10% of body weight/day. All diets were formulated by Research Diets (New Brunswick, NJ).

Following a 4-hour fast, mice were euthanized and a random lobe of liver was flash frozen in liquid nitrogen for subsequent hepatic lipid analysis. Approximately 100 mg of flash frozen liver was homogenized in a glass screw-top vial in 0.50 mL of 150 mM sodium chloride (NaCl) /5 mM 3-(N-morpholino) propanesulfonic acid (MOPS) / 1 mM EDTA/0.01% phenylmethylsulfonyl fluoride (PMSF) and extracted twice with 2:1 dichloromethane:methanol (CH₂Cl₂:MeOH). The combined bottom organic phase was concentrated to dryness at 37 °C under a stream of nitrogen and reconstituted into 95:5:5 trimethylpentane: CH₂Cl₂: MeOH (TDM). A 10 µL aliquot of the prepared sample was injected into the HPLC-ELSD system utilizing a Spherisorb® S5W Silica gel 5 µm, 100 x 4.6 mm ID HPLC column (Waters) running a 99:1 isooctane: tetrahydrofuran (mobile phase A), 2:1 acetone:CH₂Cl₂ (mobile phase B), 85:15 isopropanol:7.5 mM acetic acid/7.5 mM ethanolamine (mobile phase C) gradient before being detected using evaporative light scattering detection (ELSD) on an SEDEX® 75 detector (Sedere, Inc., Lawrenceville, NJ). Triglyceride (TG) concentrations were determined by comparing the sample peak area to the peak area of known calibration standard samples prepared in TDM.

End of study comparisons of each treatment group vs. HFD group were conducted using unpaired, two-tailed students t-test. Data was considered statistically significant at P ≤ 0.05. Results in the figure represent group mean ± SE.

### Results

Results of the experiment show that Compound A reduces hepatic triglyceride in the mice test subjects. Three week high-fat feeding resulted in increased hepatic TG levels compared to normal chow fed mice (1.6 vs. 0.6 % w/w, P = 0.03). Addition of Compound A to the high-fat diet for the final 2 weeks attenuated accumulation of hepatic TG significantly compared to high-fat feeding alone (0.6 vs. 1.6 % w/w, P = 0.02). Compound A was found to be more efficacious than Compound B (1.1 % w/w). Food consumption was similar across the HFD groups.

**Table 1**

| **Treatment Diet** | **Hepatic TG (%w/w)** | **SE** | **P-value** |
|---|---|---|---|
| NC | 0.6 | 0.2 | 0.03 |
| HFD | 1.6 | 0.4 | - |
| HFD + Compound A | 0.6 | 0.1 | 0.02 |
| HFD + Compound B | 1.1 | 0.2 | 0.22 |

## Claims

1. A compound selected from the group consisting of 1,11-bis-(1-carboxy-cyclopropyl)-undecan-6-ol and a pharmaceutically acceptable salt thereof, for use in treating a liver disorder selected from the group consisting of steatohepatitis, alcoholic liver disease, fatty liver, liver steatosis, liver cirrhosis, liver fibrosis, and acute fatty liver of pregnancy.

2. The compound for use according to claim 1, wherein the disorder is steatohepatitis.

3. The compound for use according to claim 2, wherein the steatohepatitis is nonalcoholic steatohepatitis.

4. The compound for use according to claim 2, wherein the steatohepatitis is nonalcoholic fatty liver disease.

5. The compound for use according to claim 1, wherein the disorder is alcoholic liver disease.

6. The compound for use according to claim 1, wherein the disorder is fatty liver.

7. The compound for use according to claim 1, wherein the disorder is liver steatosis, liver cirrhosis, or liver fibrosis.

8. The compound for use according to claim 1, wherein the disorder is acute fatty liver of pregnancy.

9. The compound for use according to any one of claims 1-8, wherein the compound is 1,11-bis-(1-carboxy-cyclopropyl)-undecan-6-ol.

10. A compound selected from the group consisting of 1,11-bis-(1-carboxy-cyclopropyl)-undecan-6-ol and a pharmaceutically acceptable salt thereof, for use in treating:
a. a disorder selected from the group consisting of lipodystrophy, lysosomal acid lipase deficiency, and a glycogen storage disease;
b. a disorder selected from the group consisting of hepatitis C, an infection by human immunodeficiency virus, an alpha 1-antitrypsin deficiency, Bassen-Kornzweig syndrome, hypobetalipoproteinemia, Celiac disease, Wilson disease, and Weber-Christian syndrome; or
c. a condition selected from the group consisting of toxic liver injury, total parenteral nutrition, severe surgical weight loss, environmental toxicity, malnutrition, and starvation.

11. The compound for use according to claim 10, wherein the use is for treating a disorder selected from the group consisting of lipodystrophy, lysosomal acid lipase deficiency, and a glycogen storage disease.

12. The compound for use according to claim 11, wherein the compound is 1,11-bis-(1-carboxy-cyclopropyl)-undecan-6-ol.

13. The compound for use according to claim 10, wherein the use is for a disorder selected from the group consisting of hepatitis C, an infection by human immunodeficiency virus, an alpha 1-antitrypsin deficiency, Bassen- Kornzweig syndrome, hypobetalipoproteinemia, Celiac disease, Wilson disease, and Weber-Christian syndrome.

14. The compound for use according to claim 13, wherein the compound is 1,11-bis-(1-carboxy-cyclopropyl)-undecan-6-ol.

15. A compound for use according to claim 10, wherein the use is for treating a condition selected from the group consisting of toxic liver injury, total parenteral nutrition, severe surgical weight loss, environmental toxicity, malnutrition, and starvation.

16. The compound for use according to claim 15, wherein the compound is 1,11-bis-(1-carboxy-cyclopropyl)-undecan-6-ol.

## Patentansprüche

1. Verbindung ausgewählt aus der Gruppe bestehend aus 1,11-Bis(1-carboxycyclopropyl)undecan-6-ol und einem pharmazeutisch verträglichen Salz davon zur Verwendung bei der Behandlung einer Leberstörung ausgewählt aus der Gruppe bestehend aus Steatohepatitis, alkoholischer Lebererkrankung, Fettleber, Lebersteatose, Leberzirrhose, Leberfibrose und akuter Schwangerschaftsfettleber.

2. Verbindung zur Verwendung gemäß Anspruch 1, wobei die Störung Steatohepatitis ist.

3. Verbindung zur Verwendung gemäß Anspruch 2, wobei die Steatohepatitis nicht-alkoholische Steatohepatitis ist.

4. Verbindung zur Verwendung gemäß Anspruch 2, wobei die Steatohepatitis nicht-alkoholische Fettlebererkrankung ist.

5. Verbindung zur Verwendung gemäß Anspruch 1, wobei die Störung alkoholische Lebererkrankung ist.

6. Verbindung zur Verwendung gemäß Anspruch 1, wobei die Störung Fettleber ist.

7. Verbindung zur Verwendung gemäß Anspruch 1, wobei die Störung Lebersteatose, Leberzirrhose oder Leberfibrose ist.

8. Verbindung zur Verwendung gemäß Anspruch 1, wobei die Störung akute Schwangerschaftsfettleber ist.

9. Verbindung zur Verwendung gemäß einem der Ansprüche 1-8, wobei die Verbindung 1,11-Bis(1-carboxycyclopropyl)undecan-6-ol ist.

10. Verbindung ausgewählt aus der Gruppe bestehend aus 1,11-Bis(1-carboxycyclopropyl)undecan-6-ol und einem pharmazeutisch verträglichen Salz davon zur Verwendung bei der Behandlung von:
a. einer Störung ausgewählt aus der Gruppe bestehend aus Lipodystrophie, lysosomale-saure-Lipase-Defizienz und einer Glycogenspeichererkrankung;
b. einer Störung ausgewählt aus der Gruppe bestehend aus Hepatitis C, einer Infektion durch ein humanes Immunschwächevirus, einer alpha-1-Antitrypsin-Defizienz, Bassen-Kornzweig-Syndrom, Hypobetalipoproteinämie, Zöliakie, Wilson-Erkrankung und Weber-Christian-Syndrom; oder
c. einem Zustand ausgewählt aus der Gruppe bestehend aus toxischer Leberschädigung, vollständig parenteraler Ernährung, schwerem chirurgiebedingtem Gewichtsverlust, Umwelttoxizität, Mangelernährung und Hunger.

11. Verbindung zur Verwendung gemäß Anspruch 10, wobei die Verwendung zur Behandlung einer Störung ausgewählt aus der Gruppe bestehend aus Lipodystrophie, lysosomalesaure-Lipase-Defizienz und einer Glycogenspeichererkrankung ist.

12. Verbindung zur Verwendung gemäß Anspruch 11, wobei die Verbindung 1,11-Bis(1-carboxycyclopropyl)undecan-6-ol ist.

13. Verbindung zur Verwendung gemäß Anspruch 10, wobei die Verwendung für eine Störung ausgewählt aus der Gruppe bestehend aus Hepatitis C, einer Infektion durch ein humanes Immunschwächevirus, einer alpha-l-Antitrypsin-Defizienz, Bassen-Kornzweig-Syndrom, Hypobetalipoproteinämie, Zöliakie, Wilson-Erkrankung und Weber-Christian-Syndrom ist.

14. Verbindung zur Verwendung gemäß Anspruch 13, wobei die Verbindung 1,11-Bis(1-carboxycyclopropyl)undecan-6-ol ist.

15. Verbindung zur Verwendung gemäß Anspruch 10, wobei die Verwendung zur Behandlung eines Zustands ausgewählt aus der Gruppe bestehend aus toxischer Leberschädigung, vollständig parenteraler Ernährung, schwerem chirurgiebedingtem Gewichtsverlust, Umwelttoxizität, Mangelernährung und Hunger ist.

16. Verbindung zur Verwendung gemäß Anspruch 15, wobei die Verbindung 1,11-Bis(1-carboxycyclopropyl)undecan-6-ol ist.

## Revendications

1. Composé choisi dans le groupe constitué du 1,11-bis-(1-carboxy-cyclopropyl)-undécan-6-ol et d'un sel pharmaceutiquement acceptable de celui-ci, pour utilisation dans le traitement d'un trouble hépatique choisi dans le groupe constitué d'une stéatohépatite, une hépatopathie alcoolique, une hépatite graisseuse, une stéatose hépatique, une cirrhose du foie, une fibrose hépatique et une stéatose hépatique aiguë gravidique.

2. Composé pour utilisation selon la revendication 1, le trouble étant une stéatohépatite.

3. Composé pour utilisation selon la revendication 2, la stéatohépatite étant une stéatohépatite non alcoolique.

4. Composé pour utilisation selon la revendication 2, la stéatohépatite étant une stéatose hépatique non alcoolique.

5. Composé pour utilisation selon la revendication 1, le trouble étant une hépatopathie alcoolique.

6. Composé pour utilisation selon la revendication 1, le trouble étant une hépatite graisseuse.

7. Composé pour utilisation selon la revendication 1, le trouble étant une stéatose hépatique, une cirrhose du foie ou une fibrose hépatique.

8. Composé pour utilisation selon la revendication 1, le trouble étant une stéatose hépatique aiguë gravidique.

9. Composé pour utilisation selon l'une quelconque des revendications 1 à 8, le composé étant le 1,11-bis-(1-carboxy-cyclopropyl)-undécan-6-ol.

10. Composé choisi dans le groupe constitué di 1,11-bis-(1-carboxy-cyclopropyl)-undécan-6-ol et d'un sel pharmaceutiquement acceptable de celui-ci, pour utilisation dans le traitement de :
a. un trouble choisi dans le groupe constitué d'une lipodystrophie, un déficit en lipase acide lysosomale et une maladie de stockage du glycogène ;
b. un trouble choisi dans le groupe constitué de l'hépatite C, une infection par le virus d'immunodéficience humaine, un déficit en alpha1-antitrypsine, le syndrome de Bassen-Komzweig, une hypobêtalipoprotéinémie, une maladie cœliaque, la maladie de Wilson et le syndrome de Weber-Christian ; ou
c. une affection choisie dans le groupe constitué d'une lésion hépatique toxique, une nutrition parentérale totale, une perte de poids chirurgicale sévère, une toxicité environnementale, une malnutrition et une inanition.

11. Composé pour utilisation selon la revendication 10, l'utilisation étant pour traiter un trouble choisi dans le groupe constitué d'une lipodystrophie, un déficit en lipase acide lysosomale et une maladie de stockage du glycogène.

12. Composé pour utilisation selon la revendication 11, le composé étant le 1,11-bis-(1-carboxy-cyclopropyl)-undécan-6-ol.

13. Composé pour utilisation selon la revendication 10, l'utilisation étant pour un trouble choisi dans le groupe constitué de l'hépatite C, une infection par le virus d'immunodéficience humaine, un déficit en alpha1-antitrypsine, le syndrome de Bassen-Komzweig, une hypobêtalipoprotéinémie, une maladie cœliaque, la maladie de Wilson et le syndrome de Weber-Christian.

14. Composé pour utilisation selon la revendication 13, le composé étant le 1,11-bis-(1-carboxy-cyclopropyl)-undécan-6-ol.

15. Composé pour utilisation selon la revendication 10, l'utilisation pour traiter une affection choisie dans le groupe constitué d'une lésion hépatique toxique, une nutrition parentérale totale, une perte de poids chirurgicale sévère, une toxicité environnementale, une malnutrition et une inanition.

16. Composé pour utilisation selon la revendication 15, le composé étant le 1,11-bis-(1-carboxy-cyclopropyl)-undécan-6-ol.
